# EUROPÄISCHE PATENTSCHRIFT

(11) **EP 4 422 576 B1**
(45) Veröffentlichungstag und Bekanntmachung des Hinweises auf die Patenterteilung: **25.03.2026**
(21) Anmeldenummer: 22813129.8
(22) Anmeldetag: 27.10.2022
(51) Int. Cl.: A61H 15/00, A61H 15/02, A61H 23/00, A61H 13/00, A61H 21/00

(54) **LOGOPÄDISCHES BEHANDLUNGSGERÄT**
LOGOPAEDIC TREATMENT DEVICE
DISPOSITIF DE TRAITEMENT LOGOPÉDIQUE

(30) Priorität: 28.10.2021 DE 102021128188
(43) Veröffentlichungstag der Anmeldung: 04.09.2024
(73) Patentinhaber: CODONIS AG, 4132 Muttenz (CH)
(72) Erfinder: STÜBINGER, Stefan, 4102 Binningen (CH); CIRILLO, Fabio, 4466 Ormalingen (CH)
(74) Vertreter: Specht, Peter
(86) Internationale Anmeldenummer: PCT/EP2022/080133
(87) Internationale Veröffentlichungsnummer: WO 2023/073132

(56) Entgegenhaltungen:
- WO-A1-2019/144747
- WO-A1-91/03215
- WO-A2-2009/060456
- JP-A- H1 133 069

## Beschreibung

### Technisches Gebiet

Die vorliegende Erfindung betrifft ein logopädisches Behandlungsgerät, auch bekannt und nachfolgend benannt als LWZ-Trainer (Lippen Wangen Zunge).

### Stand der Technik

Die Entwicklung des LWZ-Trainers geht zurück auf die langjährige Forschungsarbeit von Dr. h.c. Codoni. Der LWZ-Trainer wird als Hilfsmittel in der Logopädie, Zahnmedizin, der Kieferorthopädie, der Pädiatrie, der HNO, der Phoniatrie und der Mund-, Kiefer- und Gesichtschirurgie eingesetzt.

Vorwiegend wird durch den LWZ-Trainer das Training der Zungenlage und des Lippenschlusses geübt. Auch bei orofacialen Störungen, zur Unterstützung einer gesunden Nasenatmung, zum Abgewöhnen verschiedener Habits, zur Stabilisierung der Funktion der mimischen Muskulatur, zur Reduktion von Hypersalivation, und zur Verbesserung der Aussprache von Primär-Lauten wie S, SCH und Z resp. Sekundärlauten wie L, N, D und T kann der Trainer in bereits bekannter Weise eingesetzt werden.

Der bekannte LWZ-Trainer ist in der Form einer Gebissschiene aus elastischem Silikon hergestellt. Eine Grundform kann durch Anpassung wie Zuschneiden oder Schleifen in die Form gebracht werden.

Eine alternative Form eines LWZ-Trainers in Erweiterung des LWZ-Trainers von Dr. h.c. Codoni wird in der DE 20 2017 000 128 U1 offenbart.

Weiterhin ist die US 2020/0 229 750 A1 aus dem Stand der Technik bekannt. Dabei handelt es sich um ein rein am Oberkiefer orientiertes und teilweise fixiertes Trainingsgerät, welches lediglich eine Stimulation der angrenzenden anatomischen Oberkieferanteile ermöglicht. Beim Öffnen des Mundes besteht die Gefahr eines unbeabsichtigten Lösens des Trainingsgeräts, was als störend und ablenkend empfunden wird. Zudem kann das Gerät dadurch leicht mit der Zunge ungewollt manipuliert werden

Die US 2020/0 215 384 A1 weist im Lippenschlussbereich einen massiven Vorsprung auf der Aufbissebene auf, die einen funktionellen Lippenschluss beeinträchtigt. Zudem sind kleinere Vorsprünge mit Bezugszeichen 38 zur alleinigen und ausdrücklichen Stimulation des musculus mentalis unterhalb der Aufbissebene angeordnet. Die Stimulation beschränkt sich damit aufgrund der Position der Vorsprünge lediglich auf diese Muskelpartie im Frontbereich des Unterkiefers. Eine gesamtheitliche Stimulanz des Mundringmuskels und/oder der Muskeln beider Kiefer einschließlich der Wangenmuskulatur wird durch den Gegenstand dieser Druckschrift definitiv nicht erreicht.

Weiterer relevanter Stand der Technik ist in der WO 2019/144747 A1, der WO91/03215 A1 und der JP H11 33069 A offenbart.

Die Aufgabe der vorliegenden Erfindung liegt in der Verbesserung der myofunktionellen Stimulation im orofacialen Gesichtsbereich insbesondere der Lippen- und Zungenfunktion des LWZ-Trainers und insbesondere zum Trainieren eines Lippenschlusses. Zusammen mit einer integrierten oder externen Messung und Auswertung von Sensoren-Daten sollen personalisierte Therapien und deren Erfolge abgeleitet werden können. Zusätzlich ist eine Wirkstoffabgabe durch den LWZ möglich.

### Offenbarung der Erfindung

Die vorliegende Erfindung löst die vorliegende Aufgabe den LWZ-Trainer mit den Merkmalen des Anspruchs 1.

Ein erfindungsgemäßes logopädisches Behandlungsgerät weist die U-förmige Grundform einer Aufbiss-Schiene auf. Sie dient zum Einsatz in den Mund. Das logopädische Behandlungsgerät weist ein Schildsegment zum bereichsweisen Überdecken einer Zahnreihe, insbesondere der Zahnreihen des Ober- und Unterkiefers, auf. Das logopädische Behandlungsgerät weist zudem zumindest eine Beißleiste zum Aufbiss der Zähne des Ober- und Unterkiefers auf.

Bevorzugt weist die Beißleiste in Kombination mit dem Schildsegment die Möglichkeit einer modularen Anpassung an verschieden große Ober- und Unterkiefersituationen auf.

Erfindungsgemäß weist das Schildsegment auf der zahnabgewandten Seite, also in Richtung der Lippen, zumindest einen Vorsprung auf. Dabei kann es sich insbesondere um eine Anordnung mehrerer Vorsprünge handeln. Der Vorsprung oder die Anordnung mehrerer Vorsprünge dienen zur Stimulation der Lippen respektive Wangen/Gesichtsmuskulatur

Durch die Stimulation der Lippen respektive Wangen/Gesichtsmuskulatur kann ein bereits bekanntes logopädisches Behandlungsgerät - ein sogenannter LWZ-Trainer - um weitere Funktionen ergänzt werden.

Vorteilhafte Ausgestaltungen der Erfindung sind Gegenstand der Unteransprüche.

Das Schildsegment kann vorteilhaft zumindest 5, vorzugsweise zumindest 10 Vorsprünge zur Lippenstimulation aufweisen.

Die Vorsprünge können vorzugsweise eine Höhe von zumindest 0,5 mm, vorzugsweise grösser 2,0 mm aufweisen.

Ebenfalls vorzugsweise können die Vorsprünge zylindrisch, kalotten- oder kegelstumpfförmig ausgebildet sein.

Die Vorsprünge können zur weitergehenden Verbesserung der Lippenstimulanz bewegliche, insbesondere rotierbar oder linear-bewegbar gelagerte, Elemente aufweisen.

Die Vorsprünge können zur Überwachung des fortschreitenden Trainingserfolgs und/oder des Gesundheitszustands im Mund Sensorelemente aufweisen. Zur Unterstützung des Trainings kann das Behandlungsgerät zudem Aktuatoren aufweisen.

Weiterhin kann das logopädische Behandlungsgerät eine Haltefläche oder auch Vertiefung zur klemmenden oder rastenden Halterung eines Wirkstoffdepots oder eine Geschmacksstoffdepots, insbesondere eines plattenförmigen Wirkstoff- oder Geschmacksstoffdepots, aufweisen. Somit kann das logopädische Behandlungsgerät sowohl als Applikator als auch zur Stimulation dienen. So wird der Wirkstofftransport durch Anregung der Durchblutung und Speichelflusses verbessert und/oder der Geschmackssinn zusätzlich angeregt.

Das logopädische Behandlungsgerät, insbesondere die Vorsprünge können alternativ oder zusätzlich einen Wirkstoff und/oder einen Geschmacksstoff und/oder einen Indikatorstoff und/oder einen Biomarker, aufweisen.

Das logopädische Behandlungsgerät weist zudem erfindungsgemäß einen teleskopartigen Auszug zur Adaptierung des logopädischen Behandlungsgeräts an die Größe eines Gebisses auf. Der Auszug kann beispielsweise die Beißleiste und/oder das Schildsegment betreffen.

Das logopädische Behandlungsgerät kann einstückig, insbesondere monolithisch, ausgebildet sein.

Alternativ kann das logopädische Behandlungsgerät modular aufgebaut sein. Besonders bevorzugt sind dabei der oder die Vorsprünge austauschbar als Wechselmodule am Schildsegment angeordnet. Alternativ sind auch andere Elemente, wie z.B. die Beißleiste oder weitere Vorsprünge austauschbar oder extendierbar ausgebildet sein.

Das logopädische Behandlungsgerät kann zumindest bereichsweise aus einem Material mit einer Härte von Shore A 40 - 80 haben, vorzugsweise unter 70.

Vorteilhaft kann das Schildsegment, insbesondere jenseits der Vorsprünge und besonders bevorzugt auf der zahnzugewandten Seite des Schildsegments, Bereiche mit unterschiedlicher Oberflächenrauhigkeit aufweisen.

### Kurze Beschreibung der Zeichnungen

Nachfolgend wird die Erfindung anhand von Ausführungsbeispielen unter Bezug auf die Zeichnung näher beschrieben, wobei auch weitere vorteilhafte Varianten und Ausgestaltungen diskutiert werden. Es sei betont, dass die nachfolgend diskutierten Ausführungsbeispiele die Erfindung nicht abschließend beschreiben sollen, sondern dass auch nicht dargestellte Varianten und Äquivalente realisierbar sind und unter die Ansprüche fallen. Es zeigt:
- Fig. 1: Perspektivansicht eines erfindungsgemäßen LWZ-Trainers;
- Fig. 2: perspektivische Rückansicht des LWZ-Trainers;
- Fig. 3: Frontansicht des LWZ-Trainers;
- Fig. 4: Draufsicht auf den LWZ-Trainer;
- Fig. 5: Seitenansicht auf den LWZ-Trainer

### Ausführungsform(en) der Erfindung

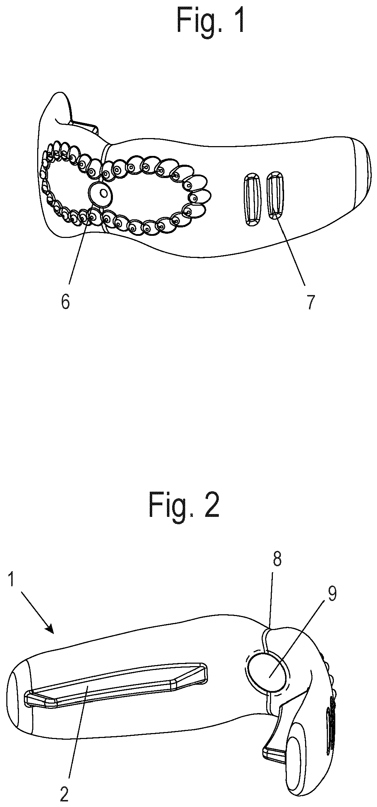

Fig. 1 zeigt ein logopädisches Behandlungsgerät. Ein solches Gerät ist in Fachkreisen auch als LWZ-Trainer 1 (Lippen Wangen Zungen - Trainer) bekannt. Das Grundbehandlungsgerät wurde von Frau Dr. h.c. Codoni entwickelt und befindet sich seit mehreren Jahren erfolgreich auf dem Markt.

Der LWZ-Trainer 1 weist eine U-förmige Grundform mit einem Schildsegment 3 und zwei in die U-Form hineinragende Beißleisten 2 auf. Die Beißleisten 2 dienen zur beidseitigen Auflage von Zähnen. Im Rahmen der vorliegenden Erfindung wird die Seite des Schildsegments 3 von welcher die Beißleisten hervorstehen als "zahnseitig" beschrieben und die dieser Seite entgegengesetzte Seite des Schildsegments als "lippenseitig" beschreiben.

Der LWZ-Trainer 1 besteht aus einem flexiblen Kunststoffmaterial, insbesondere aus einem Thermoplast und/oder aus einem thermoplastischen Elastomer.

Das Schildsegment kann umlaufend einen entformungsbedingten Grat von weniger als 0,3 mm, vorzugsweise weniger als 0,2 mm, aufweisen. Alternativ kann dieser Grat alerdings auch in einem Nacharbeitungsschritt abgetragen sein und eine entsprechende Materialabriebstelle aufweisen.

Ausgehend von dieser Grundform besteht die Weiterentwicklung des LW-Trainers in mehreren Elemente zur Verbesserung der Lippenstimulanz.

Der LWZ-Trainer weist einen oder mehrere Vorsprünge 5 zur Lippenstimulanz auf, welche aus dem Schildsegment 3 hervorstehen. Diese Vorsprünge 5 sind vorzugsweise in zumindest einer geraden oder gekrümmten Reihe angeordnet.

Die Vorsprünge 5 können jeweils tropfenförmig, ovaloid und/oder zylindrisch ausgebildet sein und jeweils eine mittige Ausnehmung aufweisen. Die reihenweise Anordnung der Vorsprünge 5 kann in einer ringförmigen Grundform 10 erfolgen.

Die Anordnung 10 mehrerer Vorsprünge 5 kann an einer Haltefläche 4 des LWZ-Trainers 1 zudem eine nicht-dargestellte Sensoranordnung und/oder ein nichtdargestelltes Wirkstoffsystem, beispielsweise jeweils in plattenförmiger Ausführung, klemmend, haftend, hakend und/oder rastend halten. Hierfür sollte die Plattenform vorzugsweise eine geringere Plattenstärke aufweisen als die Höhe zumindest einiger der Vorsprünge 5. Darüber hinaus empfiehlt sich eine Biegbarkeit der Plattenform zur besseren, vorzugsweise flächigen, Auflage auf der Haltefläche 4.

Die Haltefläche 4 kann alternativ oder zusätzlich zur Halterung einer Sensoranordnung und/oder eines Wirkstoffsystems der Anordnung einer Beschriftung dienen.

Weiterhin weist das Schildsegment 3 lippenseitig in zentraler Anordnung am Schildsegment 3 eine punktförmige Zentralfläche 6 auf. Diese kann beispielsweise durch einen Anspritzpunkt herstellungsbedingt ausgeformt sein und unterteilt das Schildsegment 3 in zwei Flügel. Die Zentralfläche 6 dient der Überprüfung der Ausrichtung und des Sitzes des LWZ-Trainers im Mund.

Zahnseitig weist das Schildsegment 3 ebenfalls eine Zentralfläche 9, vorzugsweise in punkt- oder oval-förmiger Gestalt, auf. Dabei weist diese Zentralfläche eine Kalotten- oder Kegelform auf und kann eine zu den Nachbarflächen, insbesondere der Flügel des Schildsegments eine andere Oberflächenbeschaffenheit, vorzugsweise eine andere Oberflächerauhigkeit, aufweisen.

Dies dient zur Fixierung einer Vorrichtung zur Zungenstimulanz, um eine höhere Akzeptanz des LWZ-Trainers 1 beim Nutzer, insbesondere auch bei Kindern und Jugendlichen zu schaffen.

Die jeweiligen Flügel des Schildsegments 3 weisen weitere Vorsprünge 7 auf. Alternativ können auch Einkerbungen oder Ausnehmungen vorgesehen sein. Diese dienen der besseren Greifbarkeit des LWZ-Trainers 1 und sind vorzugsweise zumindest um 15%, besonders bevorzugt um mehr als 30%, bezogen auf die Gesamtlänge des jeweiligen Flügels, von der Mitte des LWZ-Trainers 1 entfernt, in welchem die beiden Flügel aufeinandertreffen.

Die Flügel gehen ausgehend von der zweidimensionalen Ansicht auf die U-Form des LWZ-Trainers jeweils vom Scheitelpunkt ab. Zur besseren Unterteilung der beiden Flügel des Schildsegments 3 ist eine imaginäre Trennlinie 8 in die Figuren eingezeichnet.

Weisen die Flügel Vorsprünge 7 auf, so können diese zusätzlich die Lippenstimulanz unterstützen. Im Fall von Ausnehmungen oder Einkerbungen kann der Speichelfluss vom zentralen vorderen Zwischenraum zwischen dem Schildsegment 3 und den Lippen weggeleitet und/oder abgeleitet werden.

Als bevorzugtes Material des dargestellten LWZ-Trainer kann ein Kunststoffmaterial, vorzugsweise ein weiches Material, wie z.B. Silikon, eingesetzt werden. Dabei sind vorzugsweise folgende Materialdaten vorliegend, wie z.B. eine Härte Shore A 40-80 (nach DIN ISO 7619-1), eine Zugfestigkeit von 5MPa - 15MPa (nach DIN 53504/ISO 37), eine Bruchdehnung von 100-1000% (nach DIN 53504/ISO 37) und/oder eine Weiterreisswiderstand von 10 - 20 N/mm (nach ISO 34-1 Methode B (b) (Graves))

Der dargestellte LZW-Trainer 1 kann ein- oder mehrteilig ausgebildet sein. Die einteilige, vorzugsweise monolithische, Ausbildung des Trainers hat den Vorteil, dass ein unbeabsichtigtes Lösen von Teilen und/oder ein Einatmen oder Verschlucken ausgeschlossen ist.

Alternativ kann der LWZ-Trainer 1 auch mehrteilig ausgebildet sein, so dass insbesondere die Vorsprünge 5 durch austauschbare Elemente gebildet werden. So ist es beispielsweise möglich, dass die Vorsprünge 5 als Aufnahmeöffnungen des Schildsegments aus dem vorgenannten Material mit den vorgenannten Materialdaten angeordnet sind.

Die austauschbaren Elemente für die Vorsprünge können aus einem anderen, vorzugsweise einem härteren und/oder starreren Material gebildet sein. Dadurch kann die Lippenstimulanz patientenindividuell u.a. nach Entwicklungsfortschritt des Verhaltens des Nutzers, muskulärer Entwicklung und/oder Altersgruppe des Nutzers angepasst werden. Die Anpassung kann der Nutzer selbst oder insbesondere auch Fachpersonal, wie z.B. ein Logopäde oder ein logopädische Assistenz, vornehmen.

Die nicht-austauschbare Einheit aus Schildsegment und/oder Beißleisten des LWZ-Trainers kann in diesem Fall zudem in mehreren Größen erhältlich sein, so dass sie z.B. auf ein Kinder und ein Erwachsenengebiss abgestimmt sind. Ebenfalls ist die Beissleiste so ausgestaltet, dass diese teleskopisch verlängert werden kann, um die Beissleiste an verschiedene Grössen des Kiefers anzupassen.

Die Vorsprünge 5 können bewegbare gesicherte Elemente, z.B. Kugeln, aufweisen. Dabei bilden einzelne Segmente der Vorsprünge 5 zugleich die Fassung für die Kugeln, so dass diese Formschlüssig gehalten sind. Die Sicherung stellt ein versehentliches Lösen und Verschlucken der bewegbaren Elemente sicher. Die Elemente sind insbesondere rotierbar gegenüberden unbeweglichen Teilen des LWZ-Trainers, so z.B. dem Schildsegment 3, gelagert.

Besonders bevorzugt kann weniger als 50% der Oberfläche der Kugeln oder anderer bewegbar gesicherter Elemente freiliegend gegenüber der restlichen Oberfläche der Vorsprünge.

Die bewegbare-gelagerten Elemente dienen einer weitergehenden Stimulanz der Lippen und können zugleich Bewegungsabläufe der Zunge und anderer Körperelemente im Mundbereich z.B. bei der Spracherlernung, trainieren.

Alternativ oder zusätzlich kann auch in die Vorsprünge 5 sowohl die einstückigen Variante als auch in der modularen Variante des LWZ-Trainers, insbesondere mit Ausnehmungen oder Einkerbungen anstelle der Vorsprünge 5 aufweisen, die zur Befestigung von weiteren Elementen dienen. Vorzugsweise sind dies Elemente zum Schutz der Zähne, wie zum Beispiel ein Zähneschutz im Sport, aus einem harten Material, wie zum Beispiel einem Duroplast oder Thermoplast, geformt.

Alternativ oder zusätzlich kann auch in die Vorsprünge 5 sowohl die einstückigen Variante als auch in der modularen Variante des LWZ-Trainers, insbesondere mit austauschbaren Vorsprüngen 5, ein zumindest wirkstoffhaltiges und/oder geschmackhaltiges Material, zumindest ein Sensorelement oder zumindest eine Elektrode aufweisen.

Das wirkstoffhaltige Material kann insbesondere als Wirkstoffdepot die Stimulanz unterstützen. Im Fall einer notwendigen aber schmerzhaften Anwendung kann der Wirkstoff allerdings auch ein Analgetikum oder Relaxans sein.

Es hat sich zudem gezeigt, dass ein positives geschmackliches Empfinden eine vermehrte Lippenbewegung anregt und so dass ein verstärkter Trainingseffekt erreicht wird. Ein typischer Geschmacksstoff kann z.B. ein Fruchtsäureester, Zucker oder ein anderer Süssstoff, insbesondere ein zahnfreundlicher Süssstoff, wie z.B. Xylit, welcher bekannterweise keine Karies verursacht, sein.

Die Verwendung von Sensorelementen hat den besonderen Effekt, dass z.B. bei Anwendung von Biosensoren ein Entzündungsstatus durch die Konzentration einzelner Speichelinhaltsstoffe erfasst werden kann. Eine Leitfähigkeitsmessung und/oder andere physikalische Messungen, wie z.B. eine Temperaturmessung, können die Mundraumtemperatur, den Feuchtigkeitszustand im Zwischenraum und ggf. den Umfang der Durchblutung der Lippen ermittelt werden.

Elektroden können Stimulanzsignale, wie z.B. Reizstrom, zur Förderung der Durchblutung und der Muskulatur aussenden.

Selbstverständlich können die einzelnen Elemente auch vorteilhaft miteinander kombiniert werden, so können die Simulanzsignale den Speichelfluss und/oder die Releasezeiten der Wirkstoffe anregen.

Weiterhin können die Sensorelemente ermitteln, wenn ein Wirkstoff verbraucht ist und ausgetauscht werden muss oder aber ob die Stimulanzsignale aufgrund fehlerhafter Einstellung eine Entzündung oder eine überhöhte unerwünschte Erwärmung verursachen. Gleiches gilt allerdings auch für eine Detektion des Therapieerfolgs, so weist ein stärker durchblutetes Gewebe in der Regel eine höhere Temperatur und/oder eine bessere Temperaturverteilung auf.

Im Fall von elektrisch-betriebenen Sensorelementen oder Elektrode kann innerhalb des LWZ-Trainers eine Energiespeichereinheit und/oder eine Regel und/oder Auswerteeinheit angeordnet sein, welche vorzugsweise vom Material des LWZ-Trainers ummantelt ist und damit nicht mit Speichel in Berührung kommt. Es kann aufgrund der hohen mechanischen Belastung von Elektronikbauteilen bei bestimmungsgemäßer Anwendung des LWZ-Trainers auch eine mehrschichtige Ummantelung aus Material unterschiedlicher Härten vorgesehen sein.

Sollte eine weitergehende Auswertung z.B. von Speicheldaten erwünscht sein, so kann der LWZ-Trainer 1 Sammelöffnungen aufweisen, welche eine Probenahme von Speichel erlauben. Wird der LWZ-Trainer 1 nach dem Training an eine Aufladestation gekoppelt, so kann diese Aufladestation zugleich die gesammelten Speichelproben analysieren.

Der LWZ-Trainer 1 kann an verschiedene Größen für verschiedene Gebisse angepasst werden, i.e. Kinderzähne, Wechselgebiss (von Milch- zu bleibenden Zähnen), bleibende Zähne und Prothesen. Dabei kann auch lediglich die Länge der Beißleisten 2 und 3 nach Distal angepasst werden.

Zur Verlängerung der Beißleisten ist als Teleskop-Ausführung ausgeführt, dadurch Ansetzen eines Steck-Mechanismus, durch Verschraubung, Verriegelung, Bajonett, Konus, Click- und/oder Matrizen-System und/oder besonders bevorzugt durch einen Steck-Konus mit Verriegelung.

Diese verschiedenen Größen der Verlängerungen können farblich auf die Gebissdimension abgestimmt sein

Diese Verlängerung kann weiter dazu verwendet werden um Sensorik (z.B. lateral-flow-tests), Wirkstoff-Depots, Filter und/oder sonstige Bauteile oder Chemikalien zu enthalten und/oder abzugeben.

Zusätzlich zu den Elektroden können alternativ oder zusätzlich auch andere Aktuatoren z.B. zur Stimulation durch Vibration, vorzugsweise der Muskulatur, Nervenstimulation und/oder des Drüsengewebes, z.B. der Parotis aufweisen. Allgemein können die Sensoren User-Feedback der von Aktuatoren ausgelösten Reaktionen empfangen.

Wie vorbeschrieben können in dem LWZ-Trainer an- oder eingebrachten Sensoren, Wirkstoff-Depots, Aktuatoren oder User-Feedback zusammen die Wirkung haben, e.g. Stimulation durch Vibration (Aktuator), und falls ein Biomarker (Sensorik) zugegen ist, einen Wirkstoff, insbesondere dosiert, abzugeben. Vorzugsweise können neben den Vorsprüngen 5 auch andere Elemente oder Bereiche des LWZ-Trainers zur Anordnung der vorgenannten Aktuatoren, Sensoren, Wirkstoffdepots usw. genutzt werden.

So kann auch die Verlängerung der Beißschienen 2 für Wirkstoff-Depots oder andere Elemente verwendet werden, die zum Beispiel durch folgende Mechanismen ausgelöst werden:
- Mechanisch-physikalisch
- Elektrisch
- Chemisch
- Vorzugsweise: durch physikalische Einwirkung auf die Verlängerung

Die Wirkstoffe können dabei so in den Körper gelangen, dass verschieden Abgabemechanismen vorliegen können. Dies kann für chronische Schmerzen ein latentes Herauslösen bzw. "leaching" durch Osmose oder Migration sein, oder für akute Behandlungen eine Abgabe bzw. "leaching" via poröse Öffnungen durch Kapillarkräfte und/oder Potentialausgleich abgegeben werden.

Der LWZ-Trainer 1 kann dabei lippenseitig fest integrierte oder austauschbare Elemente für den Zahnschutz, Geweberegeneration, Wirkstoffabgabe, Sensorik (z.B. um Umgebungsluft zu analysieren und/oder Aktuatoren (z.B. durch Vibration zur Stimulation der Lippen oder Backen) aufweisen.

Zahnseitig können fest integrierte oder austauschbare Elemente für die Zahnfärbung, Regeneration von Zahnschmelz und Zahnfleisch, Sensorik und/oder zur Wirkstoffabgabe angeordnet sein.

Zusätzlich können am Basiskörper weitere Funktionen angebracht werden, vorzugsweise durch elektrische Kontaktelemente, Folien oder Bedruckung, die Daten zum Gesundheitszustand aufnehmen können.

Insbesondere können dies mechanische, elektrochemische, physikalische und/oder biologische Sensoren und/oder Sensorelemente sein, die über einen definierten Zeitraum in einem definierten Intervall automatisch Daten aufzeichnen.

Da solche Sensoren, aktive Sensoren mit Strombedarf sind, kann der Basiskörper weiter eine Energiequelle, Energiezelle oder Energie-"harvesting"-Device beinhalten.

Vorzugsweise sind das passive, elektrochemische Zellen, Thermo-elektrische Elemente und/oder induktions-geladene Akkumulatoren oder kapazitive Elektrospeicher. Gerade bei Elementen, die in der Art von Intarsien am oder im LWZ-Trainer 1 angeordnet sind, können diese, nach Bedarf, ausgewechselt und in den LWZ-Trainer eingesetzt werden.

Zusätzlich können die Sensoren direkt mit geeigneten externen Geräten gekoppelt werden um mittels Sensorik eine Steuerung, Schallwellenleitung und/oder Programme anzusprechen.

Zusätzlich kann die externe Basisstation zur Aufbewahrung des LWZ' dienen, als auch zum Aufladen des LWZ, oder/und auslesen der Sensorik. Weiter kann die externe Basisstation die Daten an einen zentralen Speicher übermitteln, e.g. Cloud-Speicher. oder/und Relais-Station, wie Smartphones. Vorzugweise dient die externe Basisstation als Aufbewahrungsbox und Akkustation während Reisen.

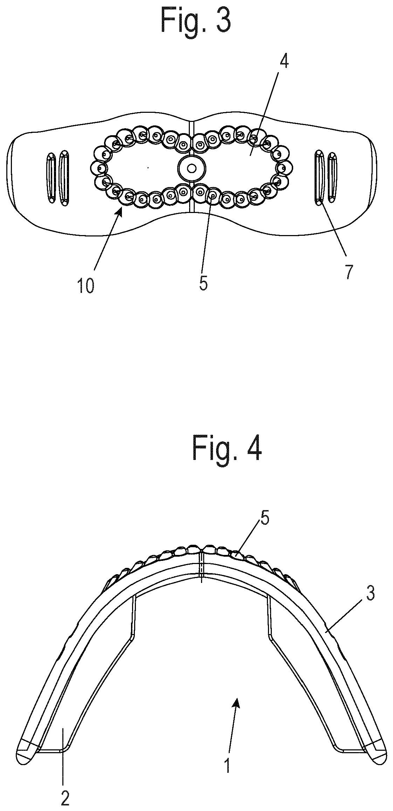

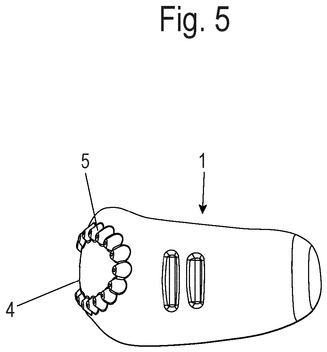

Wie aus Fig. 1-5 ersichtlich, erstreckt sich das Schildsegment 3 sowohl über die Frontzähne des Ober- und des Unterkiefers. Anders als bei einer reinen Ober- oder Unterkieferfixierung, ermöglicht die Variante der beidseitigen Fixierung durch die Ober- und Unterkieferzähne auch ein Öffnen des Mundes, wobei der Schild als Führung für die Zähne dient. Dadurch kann der erfindungsgemäße LWZ-Trainer beim Sprechen oder anderen Tätigkeiten im Mundraum sicher gehalten werden.

Die Beißleiste 2 liegt auf einer Aufbissebene, welche vorzugsweise mittig durch das Schildsegment verläuft. Die Vorsprünge 5 liegen sowohl oberhalb als auch unterhalb der Aufbissebene auf dem Schildsegment.

Dadurch werden nicht nur einzelne Muskelpartien des Ober- oder Unterkiefermuskel trainiert, sondern insbesondere der gesamte musculus orbicularis oris, also der Mundringmuskel, trainiert.

Durch das gesamtheitliche Training der Ober- und Unterlippenmuskulatur sowie der Wangenmuskeln können sowohl hängende Oberlippen oder Unterlippen aktiviert werden und auch der gesamte Lippenschluss trainiert werden.

Der LWZ-Trainer weist optional eine nicht-dargestellte Lichtquelle, z.B. eine UV-Lichtquelle, vorzugsweise in Form einer LED-Lichtquelle, auf. Diese kann z.B. für Zahnbleeching genutzt werden. Eine entsprechende Stromversorgungsquelle, wie z.B. Batterie oder ein Stromanschluss kann vorgesehen sein.

Es können andere Sensoren oder Aktuatoren im LWZ-Trainer integriert sein. Weiterhin ist ein Sende- und/oder Empfangsmodul vorzugsweise im LWZ-Trainer integriert, welches eine Verbindung mit einem externen Gerät z.B. einem Smartphone zur Steuerung der Aktuatoren, z.B. der Lichtquelle und/oder zur Auswertung der Sensordaten herstellen kann.

### Bezugszeichen

- 1: LWZ-Trainer
- 2: Beißleiste
- 3: Schildsegment
- 4: Haltefläche
- 5: Vorsprung
- 6: Zentralfläche
- 7: Vorsprung
- 8: Trennlinie
- 9: Zentralfläche
- 10: Anordnung mehrerer Vorsprünge

## Patentansprüche

1. Logopädisches Behandlungsgerät (1) mit U-förmiger Grundform zum Einsatz in den Mund, wobei das logopädische Behandlungsgerät (1) ein Schildsegment (3) zum bereichsweisen Überdecken der Zahnreihen des Ober- und Unterkiefers, aufweist und wobei das logopädische Behandlungsgerät (1) zumindest eine Beißleiste (2) zum Aufbiss der Zähne des Ober- und Unterkiefers aufweist, wobei das Schildsegment (3) auf der zahnabgewandten Seite zumindest einen Vorsprung (5), insbesondere eine Anordnung (10) mehrerer Vorsprünge (5), zur Lippenstimulation aufweist;
wobei die Beißleiste (2) eine Aufbissebene definiert und wobei die Vorsprünge (5) sowohl oberhalb als auch unterhalb der Aufbissebene angeordnet sind;
**dadurch gekennzeichnet, dass** das logopädische Behandlungsgerät (1) einen teleskopartigen Auszug zur Adaptierung des logopädischen Behandlungsgeräts (1) an die Größe eines Gebisses aufweist.

2. Logopädisches Behandlungsgerät nach Anspruch 1, **dadurch gekennzeichnet, dass** das Schildsegment (1) zumindest 5, vorzugsweise zumindest 10 Vorsprünge (5) zur Lippenstimulation aufweist.

3. Logopädisches Behandlungsgerät nach Anspruch 1 oder 2, **dadurch gekennzeichnet, dass** der oder die Vorsprünge (5) eine Höhe von größer als 0,5 mm aufweisen und vorzugsweise zylindrisch oder kegelstumpfförmig ausgebildet sind.

4. Logopädisches Behandlungsgerät nach einem der vorhergehenden Ansprüche, **dadurch gekennzeichnet, dass** der Vorsprung oder die Vorsprünge (5) bewegliche Elemente aufweisen.

5. Logopädisches Behandlungsgerät nach einem der vorhergehenden Ansprüche, **dadurch gekennzeichnet, dass** der Vorsprung **oder** die Vorsprünge (5) Sensorelemente und/oder Aktuatoren aufweist.

6. Logopädisches Behandlungsgerät nach einem der vorhergehenden Ansprüche, **dadurch gekennzeichnet, dass** das logopädische Behandlungsgerät (1) eine Haltefläche (4) zur klemmenden oder rastenden Halterung eines Wirkstoffdepots oder Geschmacksstoffdepots, insbesondere eines plattenförmigen Wirkstoffdepots oder Geschmacksstoffdepots, aufweist.

7. Logopädisches Behandlungsgerät nach einem der vorhergehenden Ansprüche, **dadurch gekennzeichnet, dass** das logopädische Behandlungsgerät (1), insbesondere die Vorsprünge (5), einen Wirkstoff und/oder einen Geschmacksstoff und/oder einen Indikatorstoff, aufweist.

8. Logopädisches Behandlungsgerät nach einem der vorhergehenden Ansprüche, **dadurch gekennzeichnet, dass** das logopädische Behandlungsgerät (1) einstückig, insbesondere monolithisch, ausgebildet ist.

9. Logopädisches Behandlungsgerät nach einem der vorhergehenden Ansprüche, **dadurch gekennzeichnet, dass** das logopädische Behandlungsgerät (1) modular aufgebaut ist, wobei besonders bevorzugt der oder die Vorsprünge (5) austauschbar am Schildsegment (3) angeordnet sind.

10. Logopädisches Behandlungsgerät nach einem der vorhergehenden Ansprüche, **dadurch gekennzeichnet, dass** das logopädische Behandlungsgerät (1) zumindest bereichsweise aus einem Material mit einer Härte von Shore A 40 - 80, vorzugsweise unter 70, ausgebildet ist.

11. Logopädisches Behandlungsgerät nach einem der vorhergehenden Ansprüche, **dadurch gekennzeichnet, dass** das Schildsegment (1), insbesondere jenseits der Vorsprünge (5) und besonders bevorzugt auf der zahnzugewandten Seite, Bereiche mit unterschiedlicher Oberflächenrauhigkeit aufweist.

## Claims

1. Speech therapy device (1) having a U-shaped basic form for use in the mouth, wherein the speech therapy device (1) has a shield segment (3) for covering areas of the rows of teeth of the upper and lower jaw, and wherein the speech therapy device (1) has at least one bite bar (2) for biting down on the upper and lower jaw teeth, wherein the shield segment (3) has at least one protrusion (5) on the side facing away from the teeth, in particular an arrangement (10) of a plurality of protrusions (5), for lip stimulation;
wherein the bite bar (2) defines a bite plane and wherein the protrusions (5) are arranged both above and below the bite plane;
**characterized in that** the speech therapy device (1) has a telescopic extension for adapting the speech therapy device (1) to the size of a set of teeth.

2. Speech therapy device according to claim 1, **characterized in that** the shield segment (1) has at least 5, preferably at least 10 protrusions (5) for lip stimulation.

3. Speech therapy device according to claim 1 or 2, **characterized in that** the protrusion or protrusions (5) have a height greater than 0.5 mm and are preferably designed in a cylindrical or truncated cone-shaped manner.

4. Speech therapy device according to one of the preceding claims, **characterized in that** the protrusion or protrusions (5) have movable elements.

5. Speech therapy device according to one of the preceding claims, **characterized in that** the protrusion **or** the protrusions (5) have sensor elements and/or actuators.

6. Speech therapy device according to one of the preceding claims, **characterized in that** the speech therapy device (1) has a holding surface (4) for clamping or snapping into place an active ingredient reservoir or flavoring agent reservoir, in particular a plate-shaped active ingredient reservoir or flavoring agent reservoir.

7. Speech therapy device according to one of the preceding claims, **characterized in that** the speech therapy device (1), in particular the protrusions (5), has an active ingredient and/or a flavoring agent and/or an indicator substance.

8. Speech therapy device according to one of the preceding claims, **characterized in that** the speech therapy device (1) is designed as a single piece, in particular monolithically.

9. Speech therapy device according to one of the preceding claims, **characterized in that** the speech therapy device (1) has a modular design, wherein it is particularly preferred that the protrusion or protrusions (5) are arranged in an exchangeable manner on the shield segment (3).

10. Speech therapy device according to one of the preceding claims, **characterized in that** the speech therapy device (1) is formed at least in some areas from a material with a Shore A hardness of 40-80, preferably below 70.

11. Speech therapy device according to one of the preceding claims, **characterized in that** the shield segment (1), in particular beyond the protrusions (5) and particularly preferably on the side facing the teeth, has areas with different surface roughness.

## Revendications

1. Dispositif de traitement logopédique (1) ayant une forme générale en U destiné à être introduit dans la bouche, le dispositif de traitement logopédique (1) comportant un segment de gouttière (3) destiné à couvrir partiellement les alignements dentaires des mâchoires supérieure et inférieure et le dispositif de traitement logopédique (1) comportant au moins une barre de morsure (2) pour l'occlusion des dents des mâchoires supérieure et inférieure, le segment de gouttière (3) présentant, du côté opposé aux dents, au moins une saillie (5), en particulier une disposition (10) de plusieurs saillies (5), pour la stimulation des lèvres,
la barre de morsure (2) définissant un plan de morsure et les saillies (5) étant disposées aussi bien au-dessus qu'en dessous du plan de morsure,
**caractérisé en ce que** le dispositif de traitement logopédique (1) comporte une glissière télescopique pour l'adaptation du dispositif de traitement logopédique (1) à la taille d'une denture.

2. Dispositif de traitement logopédique selon la revendication 1, **caractérisé en ce que** le segment de gouttière (1) comporte au moins 5, de préférence au moins 10 saillies (5) pour la stimulation des lèvres.

3. Dispositif de traitement logopédique selon la revendication 1 ou 2, **caractérisé en ce que** la ou les saillies (5) ont une hauteur supérieure à 0,5 mm et ont de préférence une forme cylindrique ou tronconique.

4. Dispositif de traitement logopédique selon l'une des revendications précédentes, **caractérisé en ce que** la saillie ou les saillies (5) comportent des éléments mobiles.

5. Dispositif de traitement logopédique selon l'une des revendications précédentes, **caractérisé en ce que** la saillie ou les saillies (5) comportent des éléments de capteur et/ou des actionneurs.

6. Dispositif de traitement logopédique selon l'une des revendications précédentes, **caractérisé en ce que** le dispositif de traitement logopédique (1) comporte une surface de maintien (4) pour le maintien par serrage ou enclenchement d'un dépôt de principe actif ou d'un dépôt d'agent aromatisant, en particulier un dépôt de principe actif ou un dépôt d'agent aromatisant en forme de plaque.

7. Dispositif de traitement logopédique selon l'une des revendications précédentes, **caractérisé en ce que** le dispositif de traitement logopédique (1), en particulier les saillies (5), comporte un principe actif et/ou un agent aromatisant et/ou une substance indicatrice.

8. Dispositif de traitement logopédique selon l'une des revendications précédentes, **caractérisé en ce que** le dispositif de traitement logopédique (1) est conçu d'une pièce, en particulier monolithique.

9. Dispositif de traitement logopédique selon l'une des revendications précédentes, **caractérisé en ce que** le dispositif de traitement logopédique (1) est conçu de façon modulaire, la ou les saillies (5) étant, de façon particulièrement préférée, disposées de manière interchangeable sur le segment de gouttière (3).

10. Dispositif de traitement logopédique selon l'une des revendications précédentes, **caractérisé en ce que** le dispositif de traitement logopédique (1) est conçu au moins en partie dans un matériau ayant une dureté Shore A de 40 à 80, de préférence inférieure à 70.

11. Dispositif de traitement logopédique selon l'une des revendications précédentes, **caractérisé en ce que** le segment de gouttière (1) présente, en particulier au-delà des saillies (5) et, de façon particulièrement préférée, sur le côté tourné vers les dents, des zones dont la rugosité de surface est différente.
